# EUROPEAN PATENT APPLICATION

(11) **EP 2 535 121 A1**
(43) Date of publication of application: **19.12.2012**
(21) Application number: 11425157.2
(22) Date of filing: 14.06.2011
(51) Int. Cl.: B08B 3/02, B08B 17/00, A61L 2/26

(54) **Apparatus for cleaning, Sanitization, disinfection and the like**

(71) Applicant: E' COSI' S.R.L., 47122 Forli', (Province of Forli-Cesena) (IT)
(72) Inventor: The designation of the inventor has not yet been filed
(74) Representative: Modiano, Micaela Nadia

(57) **Abstract**

An apparatus for cleaning, sanitization, disinfection and the like, particularly for trolleys for cleaning and other elements, comprising a casing (3) provided with at least one door (4) for accessing an inner space (5), which can be isolated from the outside as a result of closing the door (4) and which is adapted to accommodate at least one trolley for cleaning (2) and/or other elements. The trolley (2) and/or the other elements can be introduced into the space (5), and extracted therefrom, through the opening of the door (4). The space (5) is associated with at least one tank (6) of a sanitizing substance, so as to enable the spraying of the space (5) with the substance and achieve the consequent cleaning, sanitization and disinfection of the trolley (2) accommodated in the space (5).

## Description

The present invention relates to an apparatus for cleaning, sanitization, disinfection, and the like.

Nowadays, ever-increasing attention is paid to the cleaning and hygienic conditions of environments in which people live and/or work.

These needs are naturally even more felt where the guarantee of optimal conditions of hygiene constitutes an essential requirement for ensuring the positive outcome of the activities carried out in the environment, as for example is the case in hospitals, clinics and other health structures, where naturally dirt, bacterial contaminations etc, can place at risk the health of patients treated in these structures and subjected to various types of treatment in them.

In this context, the necessity to guarantee optimal conditions of hygiene and cleaning does not apply only to the instruments and utensils designed to come into contact with the patients, but also to all the exposed surfaces (of floors, walls and items of furniture) of the various rooms, as well as the medical personnel and every other person or object present or transiting in those rooms.

It thus seems evident that attaining the above-mentioned requirements for cleaning entails (as well as observing hygiene procedures and rules for people, medical instruments, floors etc.) paying great attention to a plurality of different and potential vehicles for dirt and bacteria, often not easily and immediately identifiable, but no less hazardous for the health of the patient.

More specifically, in addition to the medical personnel (and/or the nurses), the cleaning staff are also called on to observe safety procedures and measures that are expressly designed to ensure that the desired high standards of hygiene are met.

There are a number of problems in this context, however.

While in fact great attention is paid to the procedures followed by the cleaning staff, and also to the materials and detergents they use in order to carry them out, the same level of care is not dedicated to the trolley typically used by the cleaning staff to carry the detergents and other utensils needed for the work into the various rooms.

It must be pointed out, in fact, that the trolley is used to collect, over the course of the day, cloths already used for cleaning, the refuse contained in wastebaskets, and many other discarded items (linens, gauze, used syringes, phials, drip feeds and other empty containers, etc).

The trolley itself is thus transformed into a potential vehicle for pollutant and bacterial agents of various types, which can contaminate the trolley even when the waste is removed from it, rendering the next use of the trolley a potential source of danger.

Moreover, any cleaning of the trolley, if performed in the rooms in which the patients are hospitalized or in which the patients are subjected to treatments, can increase the amount of dirt, cause bacterial contamination or in any case be the cause of problems for the patient, since the detergents specifically used could be toxic for the patients.

Similarly, comparable problems and difficulties are also found in the cleaning of other elements, items of furniture and furnishings and fittings, which are also potential vehicles for dirt and bacteria, but the cleaning of which is often difficult or uncomfortable, and thus, frequently, ignored or in any case accomplished in an imprecise manner.

The aim of the present invention is to solve the above-mentioned problems, by providing an apparatus that enables the cleaning, sanitization, disinfection, and the like, of cleaning trolleys, items of furniture and other elements.

Within this aim, an object of the invention is to provide an apparatus that enables the cleaning, sanitization, disinfection, and the like, of cleaning trolleys and other elements, without the risk of spreading dirt and bacteria into the surrounding environment.

Another object of the invention is to provide an apparatus that enables the cleaning of trolleys and other elements in a manner that is very practical and quick, so as to enable the scheduling of cleaning interventions even if they are very frequent.

Another object of the invention is to provide an apparatus that makes it possible to carry out the operations of cleaning, sanitization, and disinfection without in any way affecting the environment in which it is installed.

Another object of the invention is to provide an apparatus that ensures a high reliability of operation.

Another object of the invention is to provide an apparatus that can be easily implemented using elements and materials that are readily available on the market.

Another object of the invention is to provide an apparatus that is low-cost and safely applied.

This aim and these and other objects which will become better apparent hereinafter are achieved by an apparatus for cleaning, sanitization, disinfection and the like, particularly for trolleys for cleaning and other elements, characterized in that it comprises a casing provided with at least one door for accessing an inner space, which can be isolated from the outside as a result of closing said door and which is adapted to accommodate at least one trolley for cleaning and/or other elements, which can be introduced into said space, and extracted therefrom, through the opening of said door, said space being associated with at least one tank of a sanitizing substance, for the spraying of the space with said substance and the consequent cleaning, sanitization and disinfection of the trolley accommodated in said space.

Further characteristics and advantages of the invention will become better apparent from the description of a preferred, but not exclusive, embodiment of the apparatus according to the invention, which is illustrated for the purposes of non-limiting example in the accompanying drawings wherein:
Figure 1 is a perspective schematic view of the cleaning apparatus according to the invention, with the access door closed;
Figure 2 is a perspective schematic view of the cleaning apparatus according to the invention, with the access door open, and a trolley accommodated in the space.

With reference to the figures, the apparatus according to the invention, generally designated with the reference numeral 1, can be used for activities of sanitization, disinfection and the like. In particular, it can be used to carry out these activities on cleaning trolleys 2 and other elements, for example designed to carry the utensils of cleaning staff in hospitals, care homes and other health facilities.

This use constitutes a preferred application of the present invention, and reference shall be made thereto hereinafter in the present discussion, but the possibility is not excluded of using (still within the scope of application claimed herein) the apparatus 1 according to the invention for the cleaning, sanitization and disinfection of trolleys 2 that are intended to be used in different environments, or for the cleaning of other, different elements.

For example, as will be better understood hereinafter, it is possible for the invention to be used for the cleaning of items of furniture, i.e. beds, chairs, bedside tables etc., present in the rooms of hospitals, care homes, and other health structures.

Moreover, the apparatus 1 can be installed and used in factory sheds and warehouses, and also in stations, shopping malls, public buildings (or more generally in any building in which it is desired to guarantee high standards of hygiene and cleaning, which are practically and easily attainable, as will be seen in the following pages, by way of the apparatus 1).

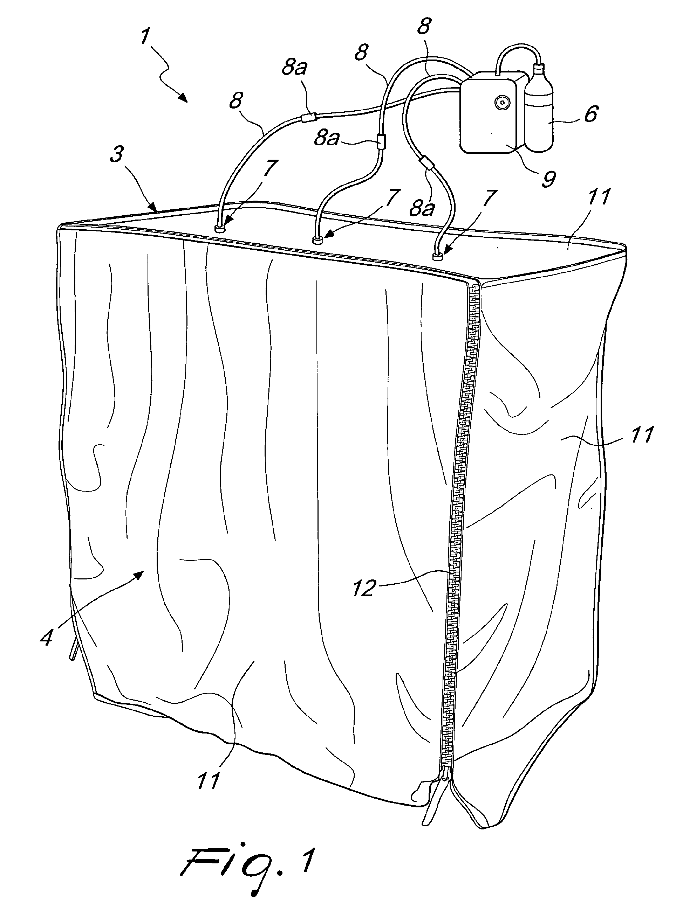

According to the invention, the apparatus 1 comprises a casing 3 provided with at least one door 4 for accessing an inner space 5, which can be isolated from the outside as a result of closing the door 4 (thus bringing the apparatus 1 into the configuration in Figure 1).

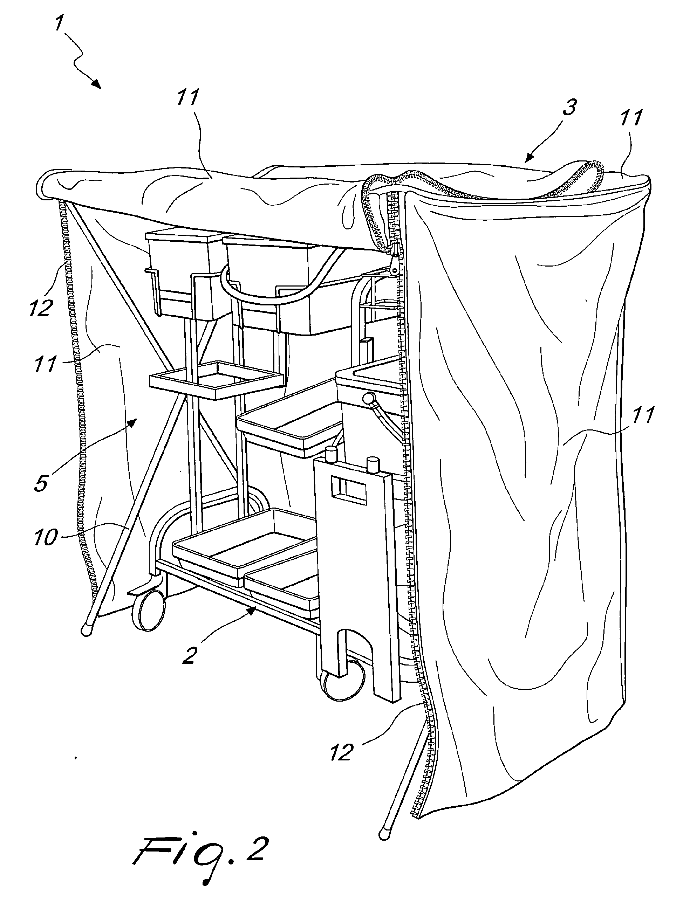

The space 5 is capable of accommodating, as can be seen in Figure 2, at least one cleaning trolley 2 (and/or another element), which can be introduced into the space 5, and extracted therefrom, by opening the access door 4.

The space 5 is associated with at least one tank 6 of a sanitizing substance, in order to enable the spraying of the space 5 with the substance and the consequent cleaning, sanitization and disinfection of the trolley 2 accommodated in the space 5.

In particular, the apparatus 1 comprises at least one nozzle 7, arranged along the casing 3 and directed toward the space 5. The nozzle 7 is connected by way of at least one connection tube 8 to the tank 6 of sanitizing substance, which, according to this configuration, which is preferred but not exclusive, is arranged outside the space 5 (as can be seen in Figure 1).

More specifically, the apparatus 1 comprises at least one valve 8a (preferably made of rubber), which is usually placed so as to close a valve area of the tube 8 (for example by way of an adjustable choke), to prevent an unwanted delivery of sanitizing substance toward the space 5.

The valve 8a can be actuated on command (for example manually by a member of staff), to open the valve area and thus enable the spraying of the space 5 with the sanitizing substance.

According to the preferred embodiment, cited for the non-limiting purpose of illustrating the application of the invention, the apparatus 1 comprises a plurality of nozzles 7, directed toward the space 5 and connected by way of respective connection tubes 8, provided with corresponding valves 8a, to the tank 6. For example, Figure 1 shows a (non-exclusive) embodiment that involves the use of three nozzles 7 associated with three respective connection tubes 8, which make it possible to distribute the sanitizing substance, optionally vaporized by the nozzles 7, more uniformly inside the space 5.

Conveniently, the apparatus 1 comprises a pump 9 (for example but not exclusively, of the volumetric type), associated with the tank 6, which can be actuated on command (also manually by a member of staff), for the automatic delivery of the sanitizing substance to the space 5, through the tubes 8 and the nozzles 7.

Advantageously, the apparatus 1 can also comprise an electronic control and management unit (an electronic controller for example), which can be reprogrammed, associated at least with the pump 9 (and optionally with the valves 8a), to command the spraying of the space 5 with the sanitizing substance.

In the electronic unit it is possible to store process parameters like the total spraying time, or the quantity of sanitizing substance to be delivered to the space 5 at each cleaning cycle, and optionally it is possible to program (and reprogram) the controller so that it is able to execute a plurality of different procedures for cleaning, sanitization, disinfection and the like, which can be selected at will by a member of staff by means of a suitable interface.

According to an embodiment of important practical interest, shown in the accompanying figures for the purposes of non-limiting example of the application of the invention, the casing 3 is substantially parallelepiped in shape and along a lateral face of this substantially parallelepiped casing 3 the door 4 for accessing the inner space 5 is defined, which can be isolated from the outside as a result of closing the door 4 substantially hermetically.

In particular, according to the preferred solution, the apparatus 1 according to the invention comprises a supporting framework 10 for a plurality of flaps 11 that are mutually coupled and made, preferably but not exclusively, of a material of the type of fabric and the like, which constitute at least the lateral faces and the top face of the substantially parallelepiped casing 3 (and optionally a further flap 11 can constitute the bottom flap, to ensure better isolation of the space 5 from the outside environment).

More specifically, the access door 4 is constituted by one of these flaps 11, which substantially forms one of the lateral faces of the casing 3 and which is coupled to the contiguous lateral faces by means of respective zip fasteners 12 (or other closure means, also of the known type, and in any case capable of ensuring the substantially hermetic isolation of the space 5). The zip fasteners 12 are arranged substantially at respective edges of the box-shaped casing 3 (and more specifically at the lateral edges of the flap 11 that constitutes the access door 4), so as to allow the opening and closing of the door 4 and access to the space 5.

Conveniently, the sanitizing substance (contained in the tank 6) is a mixture comprising hydrogen peroxide, in a percentage comprised between 0.01% and 99.99% (and preferably in a percentage comprised between 15% and 50%), and peracetic acid, in a percentage comprised between 0.01% and 99.99% (and preferably in a percentage comprised between 5% and 10%), where all the above-mentioned percentages refer to the overall weight of the mixture.

It should be noted that, while remaining within the scope of protection claimed herein, the use of other types of sanitizing substances, also of the known type, is possible, as a function of the specific applicative needs and of the standards of hygiene (and optionally of the reference standards) to be observed.

Operation of the apparatus according to the invention is apparent from the foregoing description.

More precisely, it appears evident that when it is desired to clean, sanitize and disinfect a trolley 2 (or another element) in a practical and quick manner, the apparatus 1 can be used by introducing the trolley 2 into the space 5, by opening the access door 4.

After having inserted the trolley 2, it is then possible to close the door 4 and introduce the sanitizing substance into the space 5 (for example by vaporizing/atomizing it by way of conveniently dimensioned nozzles 7), for example by operating the pump 9 (and/or the electronic control and management unit).

In this way, the substance can clean and sanitize the trolley 2, which can then be taken out and used again, by reopening the access door 4.

Similarly, it is possible to introduce other elements into the space 5, such as beds, bedside tables, chairs, other furnishings and fittings or items of furniture (usually found in the rooms of a hospital or in any case near the apparatus 1) and proceed with their cleaning, sanitization and disinfection in a practical and easy manner.

The apparatus 1 described above can easily be installed (also in multiples thereof) in one or more rooms of the building of which optimal conditions of hygiene are to be ensured (and optionally it can be provided with wheels or similar devices for enabling the transport thereof). For the member of the cleaning staff (or other designated staff), it is thus entirely practical and easy to proceed, also periodically, with the sanitization of the trolley 2, so that the latter is constantly kept clean and disinfected, thus guarding against the danger of spreading dirt and bacteria in the surrounding environment during the very transit of the trolley 2 (with any discard products collected) during the cleaning of the rooms.

For example it is possible (also as part of the procedures and rules that the member of staff is required to observe) for the apparatus 1 to be used, to carry out a cleaning cycle, every day, or after a certain number of hours of use of the trolley 2, or in any case very frequently. Also, by taking advantage of the simplicity of the required operations, it is also possible in fact for the apparatus 1 to be used after every activity of the staff member who uses the trolley 2, so as to achieve the highest guarantees of hygiene and observance of such safety rules as are in place.

It should be observed moreover that the use of a space 5 that is hermetically isolated (also by way of a careful choice of the materials that make up the casing 3 and/or the zip fasteners 12), makes it possible to guard against the danger that, during the actual activities of cleaning the trolley 2, the sanitizing substance could be spread to the outside environment, as it could be toxic to the staff of the building and/or to the member of staff who is responsible for using the apparatus 1 and the trolley 2.

Moreover, the hermetic isolation makes it possible to accommodate the trolley 2 in the space 5 even without proceeding with the sanitization, thus at the same time preventing any bacteria or other sources of contamination and dirt from being able to spread from the trolley 2 to the room. The apparatus 1 can thus be used also in order to ensure a practical and safe shelter for the trolley 2, when it is not in use.

As previously observed moreover, once the apparatus 1 is installed in the previously designated building, it will be possible to make use of it for the cleaning, sanitization and disinfection of many other elements, such as various items of furniture and furnishings and fittings. For example, but not exclusively, by way of the apparatus 1 it is possible to sanitize beds (easily introduced into the space 5 by way of the wheels with which hospital beds are typically provided), chairs, bedside tables etc. This makes it possible to guard against the danger of such elements not being thoroughly cleaned, while also offering the members of staff and of management a practical and convenient means of proceeding with thorough treatments of cleaning, sanitization and disinfection, even if they are very frequent.

The method of cleaning, sanitization, disinfection and the like, particularly for cleaning trolleys and other elements, consists first of all in a step a., in opening an access door 4 to an inner space 5 of a casing 3, of an apparatus 1. Subsequently, in a step b., the member of staff introduces into the inner space 5 at least one cleaning trolley 2 and/or other elements; with the trolley 2 accommodated in the space 5, it is possible to close, in a step c., the access door 4 in order to isolate the inner space 5 from the outside environment. At this point, the method according to the invention involves, in a step d., spraying the space 5 with a sanitizing substance coming from at least one tank 6, associated with the space 5, in order to achieve the consequent cleaning, sanitization and disinfection of the trolley 2 accommodated in said space 5. At the conclusion of the step d. described above, having thus completed the cleaning of the trolley 2, it is then possible, in a step e., to reopen the access door 4 and, in a step f., extract the trolley 2 from the space 5.

In practice it has been found that the apparatus according to the invention fully achieves the set aim, in that the use of a casing provided with at least one access door to an inner space adapted to accommodate at least one cleaning trolley and/or other elements, associated with at least one tank of a sanitizing substance, enables, by means of the spraying of the space with the substance, the cleaning, sanitization and disinfection of the trolley accommodated in the space.

The invention, thus conceived, is susceptible of numerous modifications and variations, all of which are within the scope of the appended claims. Moreover, all the details may be substituted by other, technically equivalent elements.

In the embodiments illustrated, individual characteristics shown in relation to specific examples may in reality be interchanged with other, different characteristics, existing in other embodiments.

In practice, the materials employed, as well as the dimensions, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. An apparatus for cleaning, sanitization, disinfection and the like, particularly for trolleys for cleaning and other elements, **characterized in that** it comprises a casing (3) provided with at least one door (4) for accessing an inner space (5), which can be isolated from the outside as a result of closing said door (4) and which is adapted to accommodate at least one trolley (2) for cleaning and/or other elements, which can be introduced into said space (5), and extracted therefrom, through the opening of said door (4), said space (5) being associated with at least one tank (6) of a sanitizing substance, for the spraying of the space (5) with said substance and the consequent cleaning, sanitization and disinfection of the trolley (2) accommodated in said space (5).

2. The apparatus according to claim 1, **characterized in that** it comprises at least one nozzle (7), arranged along said casing (3) and facing said space (5), said nozzle (7) being connected by means of at least one connection tube (8) to said at least one tank (6) of said sanitizing substance, arranged outside said space (5).

3. The apparatus according to claims 1 and 2, **characterized in that** it comprises at least one valve (8a), normally arranged so as to close a valve area of said tube (8), said valve (8a) being capable of being actuated on command for the opening of said valve area and enabling the spraying of said space (5) with said sanitizing substance.

4. The apparatus according to one or more of the preceding claims, **characterized in that** it comprises a plurality of said nozzles (7), facing said space (5) and connected, by means of respective connection tubes (8), provided with said corresponding valves (8a), to said at least one tank (6).

5. The apparatus according to one or more of the preceding claims, **characterized in that** it comprises a pump (9), associated with said at least one tank (6), capable of being actuated on command for the automatic delivery of said sanitizing substance to said space (5), through said tubes (8) and said nozzles (7).

6. The apparatus according to one or more of the preceding claims, **characterized in that** it comprises an electronic control and management unit, which can be reprogrammed, associated at least with said pump (9), for commanding the spraying of said space (5) with said sanitizing substance.

7. The apparatus according to claim 1, **characterized in that** said casing (3) is substantially parallelepiped in shape, said at least one access door (4) to said inner space (5) being defined along a lateral face of said substantially parallelepiped casing (3), which inner space can be isolated from the outside as a result of closing said door (4) substantially hermetically.

8. The apparatus according to one or more of the preceding claims, **characterized in that** it comprises a supporting framework (10) for a plurality of flaps (11) that are mutually coupled and made preferably of a material of the type of fabric and the like, which constitute at least the lateral faces and the top face of said substantially parallelepiped casing (3).

9. The apparatus according to one or more of the preceding claims, **characterized in that** said access door (4) is constituted by one of said flaps (11), substantially forming one of said lateral faces and coupled to the contiguous lateral faces by means of respective zip fasteners (12), arranged substantially at respective edges of said box-shaped casing (3), to allow the opening and closing of said door (4) and access to said space (5).

10. The apparatus according to claim 1, **characterized in that** said sanitizing substance is a mixture comprising hydrogen peroxide, in a percentage comprised between 0.01% and 99.99% and preferably in a percentage comprised between 15% and 50%, said percentages referring to the overall weight of the mixture, and peracetic acid, in a percentage comprised between 0.01% and 99.99% and preferably in a percentage comprised between 5% and 10%, said percentages referring to the overall weight of the mixture.

11. A method of cleaning, sanitization, disinfection and the like, particularly for trolleys for cleaning and other elements, by means of an apparatus according to one or more of the preceding claims, **characterized in that** it comprises the steps that consist in:
a. opening an access door (4) to an inner space (5) of a casing (3) of the apparatus (1);
b. introducing into said inner space (5) at least one cleaning trolley (2) and/or other elements;
c. closing said access door (4) in order to isolate said inner space (5);
d. spraying said space (5) with a sanitizing substance coming from at least one tank (6), associated with said space (5), for the consequent cleaning, sanitization and disinfection of the trolley (2) accommodated in said space (5);
e. opening said access door (4);
f. extracting the trolley (2) from said space (5).
